# EUROPEAN PATENT APPLICATION

(11) **EP 4 785 849 A1**
(43) Date of publication of application: **05.08.2026**
(21) Application number: 24871370.3
(22) Date of filing: 24.05.2024
(51) Int. Cl.: A61B 1/018, A61B 8/12, A61B 10/04, A61B 18/02

(54) **SYSTEM**

(30) Priority: 29.09.2023 JP 2023169871
(71) Applicant: FUJIFILM Corporation, Tokyo 106-8620 (JP)
(72) Inventor: OKADA, Satoru, Ashigarakami-gun, Kanagawa 258-8538 (JP); NAKAZATO, Takeharu, Ashigarakami-gun, Kanagawa 258-8538 (JP); IYAMA, Shozo, Ashigarakami-gun, Kanagawa 258-8538 (JP)
(74) Representative: HGF
(86) International application number: PCT/JP2024/019270
(87) International publication number: WO 2025/069559

(57) **Abstract**

Provided is a system that improves the efficiency of an examination or a treatment using an endoscope.

A treatment system (20) includes a tubular member (31) that has a hollow portion (31S) therein and that is provided with an ultrasound transducer (32A) at an outer periphery of the hollow portion (31S) at a distal end part (31A), and an operation handle (50) including a support member (55) that supports a proximal end part (31B) of the tubular member (31) and a connection part (51) that is connectable to a treatment tool inlet port (112) of an endoscope (1) in a state where the tubular member (31) is inserted into the treatment tool inlet port (112) from a distal end part (31A) side, in which the tubular member (31) has a proximal end surface (311) and a distal end surface (312) in an axial direction, and has a proximal end opening (3(1) 1A) connected to the hollow portion (31S) on at least the proximal end surface (311).

## Description

### BACKGROUND OF THE INVENTION

### 1. Field of the Invention

The technology of the present disclosure relates to a system used in an endoscope.

### 2. Description of the Related Art

JP2022-548739A discloses a transseptal insertion device including: a sheath defining at least one hollow portion; one or more positioning balloons connected to a distal end of the sheath; and a puncturing section movably disposed within the hollow portion.

Japanese Patent No. 4653734 discloses a medical device for inducing controlled necrosis at a target region within the body, comprising an elongated member having a proximal end part, a distal end part, and at least one hollow portion in fluid communication with a cooling fluid, a plurality of dissecting tips connected to the distal end of the elongated member and configured to contact and dissect tissue, and an ultrasound probe that controls a transmural depth of insertion of the plurality of dissecting tips into the tissue.

Japanese Patent No. 6674432 discloses a system including a probe for deploying at least one needle into tissue.

JP2021-525124A discloses an imaging component comprising a shaft comprising a proximal end, a distal end, and a cavity, and an imaging transducer connected to the distal end of the shaft.

JP2020-518385A discloses a treatment probe comprising a handle, a probe body, an imaging source connected to the probe body, and an ablation element connected to the probe body and configured to ablate a target tissue.

Japanese Patent No. 6243910 discloses a system including: an imaging component comprising an imaging handle section, an imaging shaft having a proximal end and a distal end, and an imaging transducer at the distal end of the imaging shaft; and a needle component comprising a needle handle section, a needle shaft having a distal end and a proximal end, and a needle structure reciprocably disposed on or within the needle shaft.

### SUMMARY OF THE INVENTION

The present disclosure provides the technology capable of improving the efficiency of an examination or a treatment using an endoscope.

A system according to one aspect of the technology of the present disclosure comprises a tubular member that has a hollow portion therein and that is provided, at one end part, with an ultrasound transducer at an outer periphery of the hollow portion, and a device including a first support part that supports the other end part of the tubular member and a connection part that is connectable to a treatment tool inlet port of an endoscope in a state where the tubular member is inserted into the treatment tool inlet port from a side of the one end part, in which the tubular member has a second end surface on one end side and a first end surface on the other end side in an axial direction, and has a first opening connected to the hollow portion on at least the first end surface.

A treatment method according to one aspect of the technology of the present disclosure comprises a first step of preparing a tubular member that has a hollow portion therein, the tubular member being provided, at one end part, with an ultrasound transducer at an outer periphery of the hollow portion and having an opening connected to the hollow portion on an end surface on the other end part side in an axial direction, a second step of inserting a treatment tool into the hollow portion from the opening, a third step of inserting the tubular member into which the treatment tool is inserted into a subject and aligning the ultrasound transducer with a target region of the subject based on an output of the ultrasound transducer, a fourth step of holding a position of a treatment part of the treatment tool in a state where the target region, the ultrasound transducer, and the treatment part are aligned and moving the tubular member to the other end part side to expose the treatment part from the hollow portion, and a fifth step of driving the treatment part to treat the target region after the fourth step.

According to the technology of the present disclosure, the efficiency of the examination or the treatment using the endoscope can be improved.

### BRIEF DESCRIPTION OF THE DRAWINGS

FIG. 1 is a diagram illustrating a schematic configuration of an endoscope device 100 that is one aspect of the technology of the present disclosure.
FIG. 2 is a schematic cross-sectional view illustrating a schematic configuration of a treatment system 20 that is inserted from a treatment tool inlet port 112 of an endoscope 1 illustrated in FIG. 1 into a treatment tool channel of the endoscope 1 and is used.
FIG. 3 is an enlarged view of a range X illustrated in FIG. 2.
FIG. 4 is a cross-sectional view taken along the line C-C illustrated in FIG. 3.
FIG. 5 is an enlarged view of a range Y illustrated in FIG. 2.
FIG. 6 is a diagram illustrating a state where a grip part 33 is moved to a proximal end side of a tubular member 31 from the state illustrated in FIG. 3.
FIG. 7 is a schematic diagram illustrating a modification example of a first restriction part.
FIG. 8 is a schematic diagram illustrating a state where an ultrasound probe 30 and a treatment probe 40 are disposed at an endoscope distal end part 10C.
FIG. 9 is a schematic diagram illustrating a treatment method.
FIG. 10 is a schematic diagram illustrating the treatment method.
FIG. 11 is a diagram illustrating a first modification example of the treatment system 20, and is a diagram corresponding to FIG. 3.
FIG. 12 is a diagram illustrating a second modification example of the treatment system 20, and is a diagram corresponding to FIG. 3.
FIG. 13 is a schematic cross-sectional view taken along the line D-D in FIG. 12.

### DESCRIPTION OF THE PREFERRED EMBODIMENTS

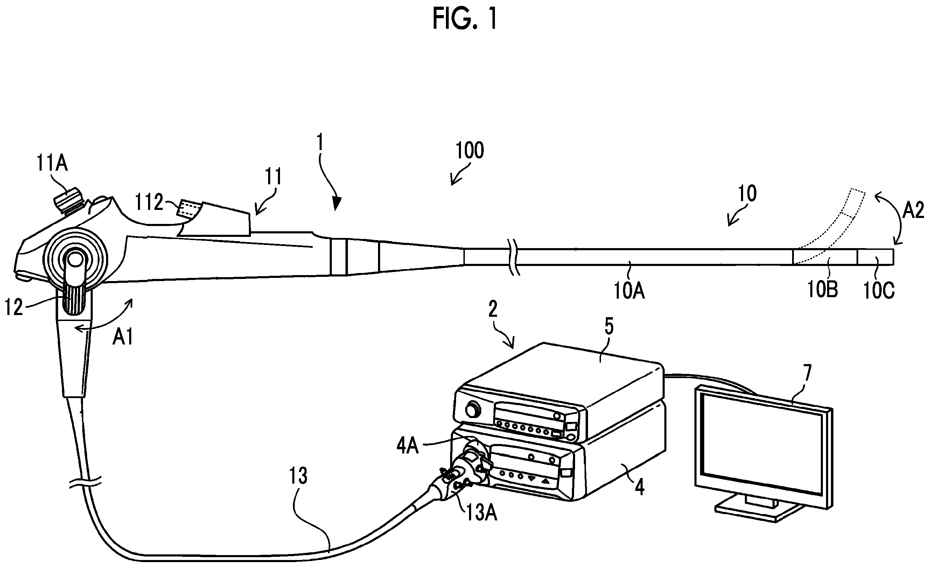

FIG. 1 is a diagram illustrating a schematic configuration of an endoscope device 100 that is one aspect of the technology of the present disclosure. The endoscope device 100 comprises an endoscope 1, a body part 2 consisting of a light source device 4 and a processor device 5 to which the endoscope 1 is connected, and a display device 7 that displays a captured image captured by the endoscope 1, and the like. The endoscope 1 is a bronchoscope in the example of FIG. 1, but the present disclosure is not limited to this.

The endoscope 1 comprises an insertion part 10 that is an elongated tool extending in one direction and is inserted into a subject, an operating part 11 that is provided at a proximal end part of the insertion part 10 and is provided with an operation member for performing an observation mode switching operation, an imaging and storage operation, a forceps operation, a suction operation, and the like, an angle lever 12 that is provided adjacent to the operating part 11, and a universal cord 13 including a connector part 13A for attachably and detachably connecting the endoscope 1 to a connector connection part 4A of the light source device 4.

The operating part 11 is provided with a treatment tool inlet port 112 into which a treatment tool for collecting a biological tissue such as cells or polyps is introduced. Although not illustrated in FIG. 1, various channels such as a treatment tool channel and a suction channel into which the treatment tool introduced from the treatment tool inlet port 112 is inserted are provided inside the operating part 11 and the insertion part 10. The operating part 11 includes a suction button 11A and the like. A suction tube (not illustrated) is connected to the suction button 11A, and the suction tube is connected to a suction device. In a case where the suction button 11A is operated, a fluid or the like is suctioned from a distal end of the endoscope 1 to the suction tube and then collected by the suction device.

The insertion part 10 includes a flexible part 10A having flexibility, a bendable part 10B provided at a distal end of the flexible part 10A, and an endoscope distal end part 10C that is harder than the flexible part 10A and is provided at a distal end of the bendable part 10B. An imaging element and an imaging optical system are built in the endoscope distal end part 10C.

The bendable part 10B is configured to perform a bending operation in an up-down direction (A2 direction) by rotationally operating the angle lever 12 of the operating part 11 in an A1 direction, and the endoscope distal end part 10C can be directed in a desired direction by performing the bending operation of the bendable part 10B.

A light guide configured by bundling a plurality of optical fibers is provided inside the endoscope 1 from the endoscope distal end part 10C of the insertion part 10 to the connector part 13A. The light generated by the light source device 4 is introduced from the connector part 13A into the light guide and travels to the endoscope distal end part 10C, and is emitted to the subject from an illumination window provided in the endoscope distal end part 10C.

The configuration of the endoscope 1 is an example, and is not limited to the configuration illustrated in FIG. 1.

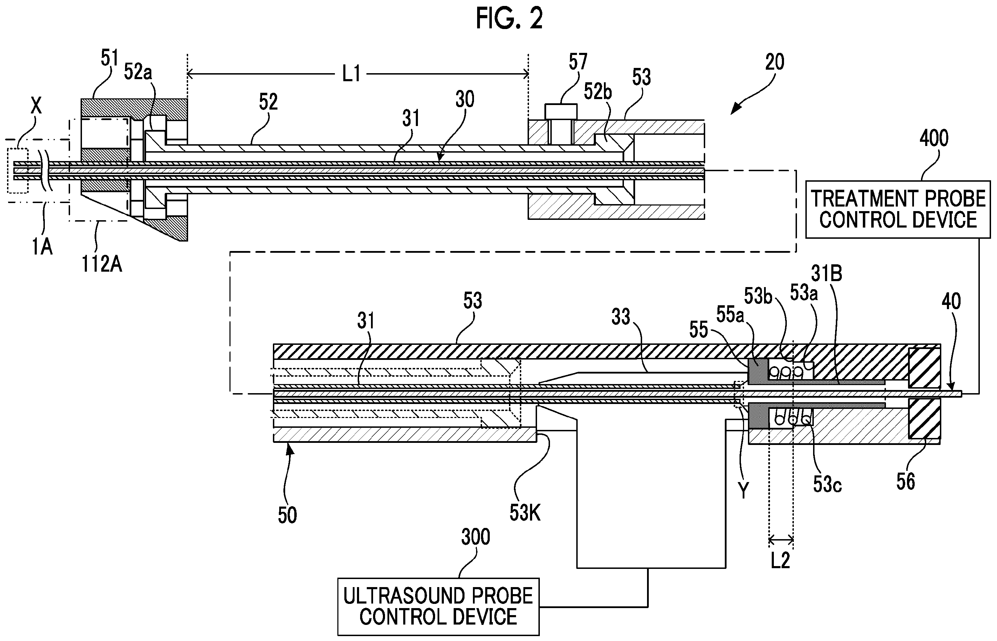

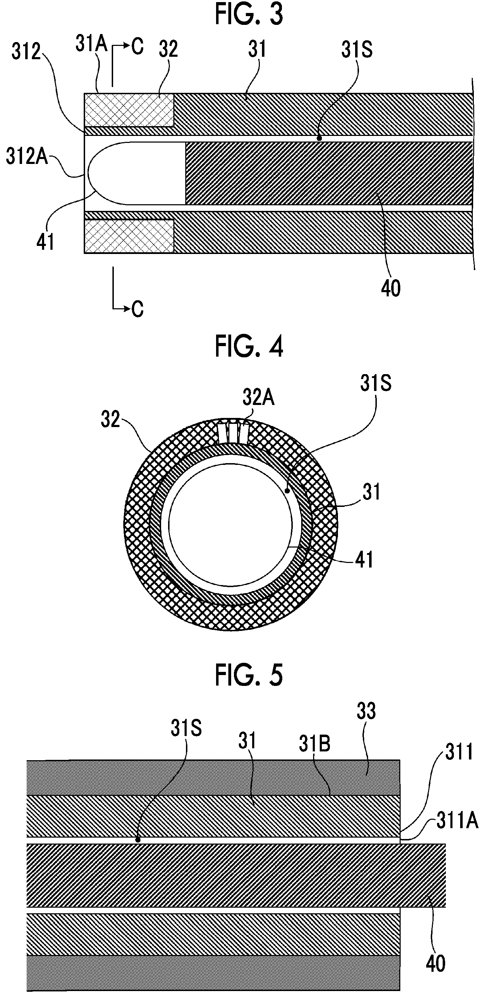

FIG. 2 is a schematic cross-sectional view illustrating a schematic configuration of the treatment system 20 that is inserted from the treatment tool inlet port 112 of the endoscope 1 illustrated in FIG. 1 into the treatment tool channel of the endoscope 1 and is used. FIG. 3 is an enlarged view of a range X illustrated in FIG. 2. FIG. 4 is a cross-sectional view taken along the line C-C illustrated in FIG. 3. FIG. 5 is an enlarged view of a range Y illustrated in FIG. 2.

The treatment system 20 comprises an ultrasound probe 30 that is an elongated tool extending in one direction, a treatment probe 40 that is an elongated treatment tool extending in one direction, and an operation handle 50 that is configured to support the ultrasound probe 30 and the treatment probe 40.

The ultrasound probe 30 comprises a tubular member 31 that is made of a resin, an elastomer, or the like and that has, for example, a cylindrical hollow portion 31S (see FIGS. 3 and 5) therein. As illustrated in FIG. 3, an ultrasound transmission/reception unit 32 is provided at an outer periphery of the hollow portion 31S at a distal end part 31A that is an end part on one end side in the axial direction of the tubular member 31. As illustrated in FIG. 4, the ultrasound transmission/reception unit 32 is preferably provided in an annular shape as viewed in the axial direction of the tubular member 31. The ultrasound transmission/reception unit 32 may not be provided in an annular shape as viewed in the axial direction of the tubular member 31, and may be provided in, for example, an arc shape (C shape, semicircular shape, or the like) as viewed in the axial direction of the tubular member 31. Since the ultrasound transmission/reception unit 32 is provided in an annular shape, the ultrasound image in the range of 360 degrees around the distal end part 31A in the subject can be easily obtained based on the echo received by the ultrasound transmission/reception unit 32.

The ultrasound transmission/reception unit 32 includes a plurality of ultrasound transducers 32A (partially illustrated in FIG. 4) that are arranged along a circumferential direction of the distal end part 31A of the tubular member 31, and an acoustic matching layer, an acoustic lens, and the like (not illustrated). A planar shape of the ultrasound transducer 32A as viewed in a vibration direction thereof is, for example, a rectangle. The ultrasound transducer 32A is provided at the distal end part 31A, for example, in a state where a longitudinal direction thereof is along the axial direction of the tubular member 31.

A wiring group electrically connected to the ultrasound transmission/reception unit 32 is provided in a wall part of the tubular member 31. The wiring group extends to the vicinity of a proximal end part 31B that is an end part on the other end side of the tubular member 31. As illustrated in FIG. 2, a substantially T-shaped grip part 33 is fixed to a vicinity of the proximal end part 31B of the tubular member 31, and the wiring group extends to the inside of the grip part 33. The wiring group is connected to an ultrasound probe control device 300 that performs control of the ultrasound transmission/reception unit 32 and generation and display of the ultrasound image.

As illustrated in FIG. 3, a distal end opening 312A connected to the hollow portion 31S is provided on a distal end surface 312 that is an end surface of the tubular member 31 on one end side in the axial direction. As illustrated in FIG. 5, a proximal end opening 311A connected to the hollow portion 31S is provided on a proximal end surface 311 that is an end surface of the tubular member 31 on the other end side in the axial direction.

The treatment probe 40 is a treatment tool that has a treatment part 41 at a distal end and that can freeze tissue of the subject by the treatment part 41. As the treatment probe 40, a treatment probe in which the treatment part 41 is made of, for example, a metal and the treatment part 41 is cooled by using the Joule-Thomson effect is preferably used. As the treatment probe 40, for example, a cryoprobe (registered trademark) can be used.

The treatment probe 40 is used in a state of being inserted into the hollow portion 31S from the proximal end opening 311A of the ultrasound probe 30. The treatment probe 40 is connected to a treatment probe control device 400 that performs various controls such as driving the treatment part 41 (injection of a gas for cooling). It is preferable that an inner diameter of the tubular member 31 is configured to be larger than an outer diameter of the treatment probe 40.

The operation handle 50 has an elongated tubular shape in which a housing space in which a part of the ultrasound probe 30 can be housed is provided. The operation handle 50 has a tubular connection part 51 that can be connected to an adapter 112A provided at the treatment tool inlet port 112 of the endoscope 1 by fitting or the like at a distal end thereof.

The operation handle 50 further includes a tubular shaft member 52 that is supported by the connection part 51 and extends to a side opposite to the connection part 51 side. In the shaft member 52, a flange part 52a is provided at an end part on the connection part 51 side, and a flange part 52b is provided at an end part on a side opposite to the connection part 51 side. The flange part 52a is inserted into the connection part 51, and is fixed inside the connection part 51. The tubular member 31 is inserted into the entire interior of the shaft member 52. The tubular member 31 on the distal end side of the flange part 52a of the shaft member 52 is inserted into the adapter 112A connected to the connection part 51 through the inside of the connection part 51.

In a case where the treatment system 20 is used, the ultrasound probe 30 led out to the outside of the connection part 51 is inserted into the treatment tool channel 1A of the endoscope 1 from the distal end part 31A through the adapter 112A, and the connection part 51 is connected to the adapter 112A in this state. The connection part 51 is connectable to the treatment tool inlet port 112 in a state where the tubular member 31 is inserted into the treatment tool inlet port 112 of the endoscope 1 from the distal end part 31A side.

The operation handle 50 further comprises a tubular slider 53 that is slidably connected to an outer peripheral surface between the flange part 52a and the flange part 52b of the shaft member 52 in the axial direction of the shaft member 52. An opening is provided on an outer peripheral surface of the slider 53 at an end part on the connection part 51 side. A fixing member 57 such as a screw for fixing (locking) a relative position of the slider 53 to the shaft member 52 is inserted into the opening. In a state where the fixing member 57 is unlocked, the slider 53 is configured to move in a range of a distance L1 between a position illustrated in FIG. 2 at which the inner wall and the flange part 52b are in contact with each other and a position at which the end surface on the connection part 51 side is in contact with the connection part 51.

An opening 53K is provided on the outer peripheral surface of the slider 53 on a proximal end side of a position at which the flange part 52b is disposed in a state where the end surface on the connection part 51 side is in contact with the flange part 52a (a position of the shaft member 52 in this state is indicated by a broken line in the drawing). The grip part 33 is disposed at a position at which the opening 53K is provided. A portion of the grip part 33 protrudes to the outside from the opening 53K. The grip part 33 is fixed to the tubular member 31.

A tubular support member 55 is provided inside the slider 53 adjacent to the proximal end side of the grip part 33. The support member 55 supports the grip part 33 or the proximal end part 31B of the tubular member 31 fixed to the grip part 33, and is fixed to the grip part 33 or the proximal end part 31B by, for example, a magnet. The support member 55 has a flange part 55a at an end part on the distal end side. An inner space of the slider 53 on the proximal end side with respect to the grip part 33 is narrowed in two stages toward the proximal end side, and a proximal end side of the support member 55 is inserted into a narrowest portion. On the inner wall of the slider 53, a step portion 53b and a step portion 53a are formed to be arranged in this order from the distal end side on the proximal end side with respect to the flange part 55a. A biasing portion 53c such as a spring that biases the flange part 55a to the distal end side is provided between the flange part 55a and the step portion 53a of the support member 55. The support member 55 is configured to be movable from an initial position illustrated in FIG. 2 to a position at which the flange part 55a is in contact with the step portion 53b. For example, by moving the grip part 33 in the right direction in FIG. 2 by the operator gripping the grip part 33, the tubular member 31 (that is, the ultrasound probe 30) fixed to the grip part 33 and the support member 55 can be moved in the axial direction of the tubular member 31 by a distance L2.

A support member 56 that supports the treatment probe 40 is provided on the proximal end side with respect to the support member 55 in the slider 53. The support member 56 is made of an annular rubber or the like that fits into the opening provided on the proximal end surface of the slider 53, and the treatment probe 40 can be inserted. The support member 56 can fix and support the inserted treatment probe 40 by tightening a screw or the like (not illustrated) to narrow the hollow portion. The treatment probe 40 inserted into the support member 56 is inserted into the hollow portion 31S of the tubular member 31 through the support member 55 and is inserted into the distal end of the tubular member 31.

In the treatment system 20, it is preferable that the ultrasound probe 30 and the grip part 33 are attachably and detachably configured to the operation handle 50. In a case where the treatment system 20 is used, first, an assembly including the ultrasound probe 30 and the grip part 33 fixed to the ultrasound probe 30 is prepared, and the assembly is inserted into the slider 53 from the opening 53K. In this case, the ultrasound probe 30 is inserted from the distal end side into the slider 53, the shaft member 52, and the connection part 51 in this order, and most of the ultrasound probe 30 is exposed to the outside from the connection part 51. In addition, the grip part 33 or the proximal end part 31B of the tubular member 31 may be fixed to the support member 55 by a magnet, adhesion, a locking structure, or the like. The treatment system 20 may have a configuration in which the operation handle 50, the ultrasound probe 30, and the grip part 33 are integrated and only the treatment probe 40 is attachable and detachable.

Next, the treatment probe 40 is inserted into the support member 56 from the treatment part 41 side, and the treatment probe 40 is inserted into the hollow portion 31S of the tubular member 31 supported by the support member 55. Then, the distal end of the treatment probe 40 is inserted into the distal end of the tubular member 31 such that the positional relationship illustrated in FIG. 3 is obtained. In the example of FIG. 3, in a state of being viewed in the radial direction of the tubular member 31, the ultrasound transmission/reception unit 32 and the treatment part 41 overlap each other, and a position of the ultrasound transmission/reception unit 32 and a position the treatment part 41 in the axial direction of the tubular member 31 are substantially the same.

In addition, as illustrated in FIG. 3, the treatment probe 40 is supported by the support member 56 in a state where the treatment part 41 is retracted into the hollow portion 31S from the distal end opening 312A. The grip part 33 fixed to the ultrasound probe 30 and the support member 55 are movable in the axial direction in the slider 53. Therefore, in a case where the grip part 33 is moved to the proximal end side by the distance L2 from the position illustrated in FIG. 2, the ultrasound probe 30 can be moved by the distance L2 with respect to the treatment probe 40.

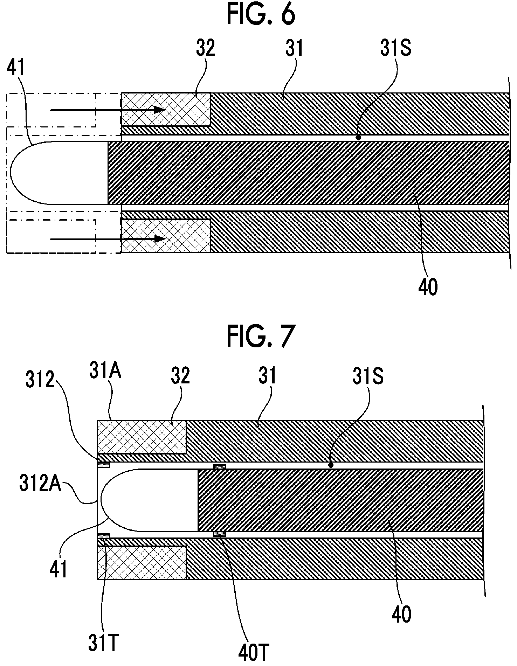

FIG. 6 is a diagram illustrating a state where the grip part 33 is moved to the proximal end side of the tubular member 31 from the state illustrated in FIG. 3. By moving the grip part 33 to the proximal end side by the distance L2 from the position illustrated in FIG. 2, as illustrated in FIG. 6, the treatment part 41 can be in a state of protruding through the distal end opening 312A to the outside of the hollow portion 31S. As described above, the support member 55 and the support member 56 support the tubular member 31 and the treatment probe 40 such that the relative position between the treatment part 41 of the treatment probe 40 and the distal end surface 312 of the tubular member 31 can be changed.

The support member 55 can be moved only between the initial position and the position in contact with the step portion 53b. That is, the protrusion amount of the treatment part 41 from the hollow portion 31S is restricted by the support member 55, the biasing portion 53c, and the step portion 53b. The support member 55, the biasing portion 53c, and the step portion 53b constitute a first restriction part.

The protrusion amount of the treatment part 41 from the hollow portion 31S can be restricted at the distal end of the ultrasound probe 30 and the treatment probe 40 instead of the operation handle 50. For example, as illustrated in FIG. 7, an annular protrusion part 31T may be provided on an inner peripheral surface of the distal end part 31A of the tubular member 31, and a protrusion part 40T that can be brought into contact with the protrusion part 31T may be provided on an outer peripheral surface of the treatment probe 40 in the vicinity of the treatment part 41. In a case where the grip part 33 is moved and the tubular member 31 is moved in the right direction in FIG. 7, since the protrusion amount of the treatment part 41 from the hollow portion 31S is maximized in a state where the protrusion part 31T and the protrusion part 40T are in contact with each other, the protrusion amount can be restricted. In the configuration illustrated in FIG. 7, the protrusion part 31T and the protrusion part 40T constitute a first restriction part.

In addition, in the operation handle 50, the position (support position of the tubular member 31) of the support member 55 may be fixed and the position (support position of the treatment probe 40) of the support member 56 may be movable such that the relative position between the treatment part 41 of the treatment probe 40 and the distal end surface 312 of the tubular member 31 can be changed. In the operation handle 50, the position of the support member 55 and the position of the support member 56 may be movable such that the relative position between the treatment part 41 of the treatment probe 40 and the distal end surface 312 of the tubular member 31 can be changed.

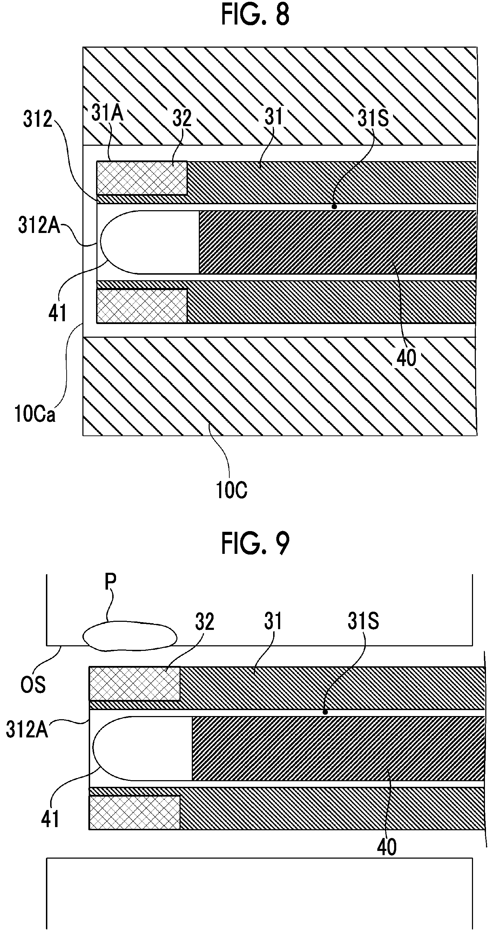

After the state (state where the ultrasound transmission/reception unit 32 and the treatment part 41 are aligned) illustrated in FIG. 3 is obtained in which the treatment probe 40 is inserted into the ultrasound probe 30, the ultrasound probe 30 in a state where the treatment probe 40 is inserted is inserted into the treatment tool inlet port 112 from the distal end side, and then the connection part 51 is connected to the treatment tool inlet port 112. In this state, as illustrated in FIG. 8, the ultrasound probe 30 and the treatment probe 40 retract into the inside of the endoscope distal end part 10C with respect to a treatment tool outlet port 10Ca formed on the end surface of the endoscope distal end part 10C.

In a case where the slider 53 is moved along the shaft member 52 in the state illustrated in FIG. 8, in a state where the grip part 33 is not moved with respect to the slider 53, since the position of the support member 55 in the slider 53 is unchanged by the biasing force of the biasing portion 53c, the ultrasound probe 30 and the treatment probe 40 can be moved at the same time. As a result, the ultrasound probe 30 and the treatment probe 40 can protrude from the treatment tool outlet port 10Ca or the ultrasound probe 30 and the treatment probe 40 can retract into the treatment tool outlet port 10Ca. As described above, the shaft member 52, the slider 53, the biasing portion 53c, the support member 55, and the support member 56 constitute a moving mechanism that moves the tubular member 31 and the treatment probe 40 in the axial direction of the tubular member 31 in a state where the relative position between the distal end surface 312 and the treatment part 41 is held.

The slider 53 can be moved only between a position in contact with the connection part 51 and a position in contact with the flange part 52b. Therefore, the protrusion amounts of the ultrasound probe 30 and the treatment probe 40 from the treatment tool outlet port 10Ca are restricted by the shaft member 52, the slider 53, and the connection part 51. The shaft member 52, the slider 53, and the connection part 51 constitute a second restriction part.

An example of a treatment method using the endoscope 1 and the treatment system 20 configured as above will be described. First, the operator inserts the insertion part 10 of the endoscope 1 into a bronchus of the subject, and moves the endoscope distal end part 10C to a desired region. Then, the ultrasound probe 30 and the treatment probe 40 of the treatment system 20 prepared as described above are inserted into the treatment tool inlet port 112, and the connection part 51 is connected to the treatment tool inlet port 112. In addition, the treatment system 20 may be mounted on the endoscope 1 before the endoscope 1 is inserted into the subject.

Next, the operator slides the slider 53 to protrude the ultrasound probe 30 and the treatment probe 40 from the treatment tool outlet port 10Ca and to advance the ultrasound probe 30 and the treatment probe 40 in the bronchus, and aligns the ultrasound transmission/reception unit 32 with the target region P of the bronchus OS while checking the ultrasound image generated based on the output of each ultrasound transducer 32A of the ultrasound transmission/reception unit 32 (see FIG. 9). In the state illustrated in FIG. 9, the target region P, the ultrasound transducer 32A, and the treatment part 41 are aligned.

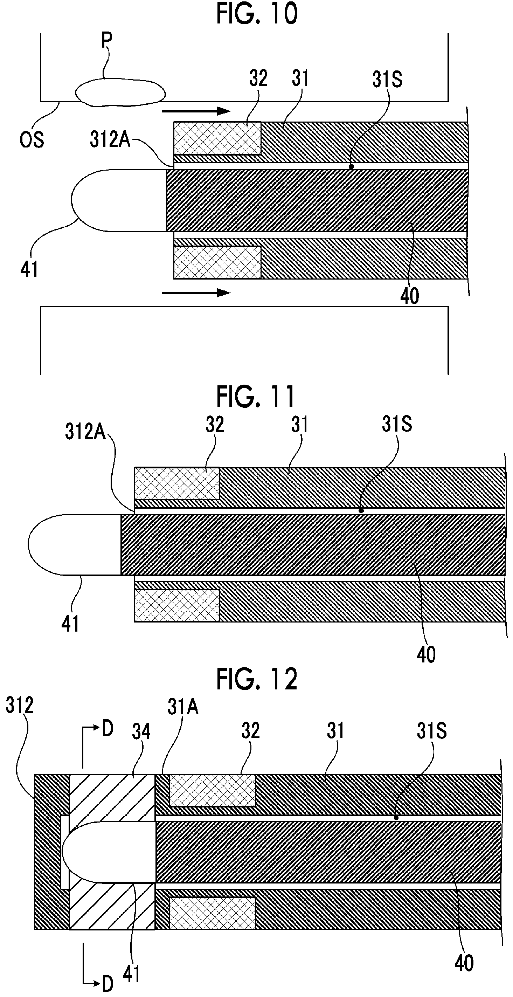

Next, in the state illustrated in FIG. 9, the operator slides the grip part 33 to the proximal end side to move the tubular member 31 to the proximal end part 31B side while holding the position of the treatment part 41, thereby exposing the treatment part 41 from the hollow portion 31S as illustrated in FIG. 10.

Next, the operator drives the treatment part 41 to treat the target region P. That is, the treatment part 41 is cooled to freeze the target region P. Thereafter, the operator removes the connection part 51 from the treatment tool inlet port 112 while maintaining the positional relationship between the ultrasound probe 30 and the treatment probe 40 illustrated in FIG. 10, and pulls out the ultrasound probe 30 and the treatment probe 40 from the treatment tool inlet port 112 to collect the frozen tissue.

As described above, with the treatment system 20, even in a narrow region such as the bronchus that cannot be observed by the endoscope 1, the target region P can be searched for by the ultrasound probe 30, and the target region P can be treated (tissue can be collected) by the treatment part 41 without removing the ultrasound probe 30 from the endoscope 1. For example, it is not necessary to perform work (work of replacing the ultrasound probe and the treatment probe) of searching for the target region P by inserting the ultrasound probe into the treatment tool channel of the endoscope 1, pulling out the ultrasound probe from the treatment tool channel, inserting the treatment probe into the treatment tool channel to advance the treatment part to the position of the target region P, and performing the treatment in this state. Therefore, the treatment can be simply and efficiently carried out.

In addition, by disposing the ultrasound transmission/reception unit 32 and the treatment part 41 at substantially the same position, the target region P and the treatment part 41 are aligned in the state illustrated in FIG. 10. Therefore, the treatment can be accurately performed on the target region P checked in the ultrasound image. In addition, compared to a method of searching for the target region using radiation, the cost of the treatment can be reduced while eliminating the influence on the subject because the ultrasound is used.

In addition, with the treatment system 20, since the ultrasound probe 30 and the treatment probe 40 can be inserted into the treatment tool channel in a state where the treatment part 41 is retracted into the hollow portion 31S, the treatment part 41 can be protected. In the example of FIG. 3, the entire treatment part 41 is retracted into the hollow portion 31S from the distal end opening 312A, but the treatment probe 40 may be supported by the support member 56 in a state where a part of the treatment part 41 is outside the hollow portion 31S. Even in this case, most of the treatment part 41 can be protected.

With the treatment system 20, since the treatment probe 40 can be inserted and pulled out with respect to the ultrasound probe 30, a commercially available treatment probe can be used as the treatment probe 40, and the introduction cost of the system can be reduced. In the treatment system 20, the operation handle 50 and the ultrasound probe 30 may be washed and reused, and the treatment probe 40 may be a single-use disposable device. Alternatively, the ultrasound probe 30 may be washed, disinfected, and reused, and the operation handle 50 and the treatment probe 40 may be a single-use disposable device.

FIG. 11 is a diagram illustrating a first modification example of the treatment system 20, and is a diagram corresponding to FIG. 3. In the modification example illustrated in FIG. 11, in a state where the ultrasound probe 30 and the treatment probe 40 are supported by the operation handle 50, the treatment part 41 protrudes through the distal end opening 312A to the outside of the hollow portion 31S. In this modification example, the support member 55 is changed to a configuration in which the support member 55 is not movable in the axial direction of the tubular member 31.

In a case where the treatment system 20 illustrated in FIG. 11 is used, after the ultrasound transmission/reception unit 32 and the target region P are aligned, the slider 53 is slightly moved to the proximal end side to move the treatment part 41 to the position where the ultrasound transmission/reception unit 32 is present, and the treatment part 41 is driven in this state to freeze the target region P.

In the modification example illustrated in FIG. 11, by providing a mechanism (for example, adding gradations or the like to the slider 53) that can move the slider 53 to the proximal end side by the same distance as the distance between the treatment part 41 and the ultrasound transmission/reception unit 32, more accurate treatment can be performed on the target region P.

The treatment probe 40 described above is not limited to the treatment probe that can freeze and collect the tissue, and the treatment probe that collects the tissue by another method can also be used. For example, the treatment part 41 can be changed to a brush-shaped treatment part. In this case, for example, in a case where the brush is folded to a degree that the brush can pass through the hollow portion 31S in a normal state and the brush is opened to a position at which the brush can contact the target region P in a case of the treatment, the insertion of the treatment probe 40 into the ultrasound probe 30 and the collection of the tissue can be achieved.

Further, the treatment part 41 can be a forceps. In this case, for example, in a case where the forceps is folded to a degree that the forceps can pass through the hollow portion 31S in a normal state and the forceps is opened to a position at which the forceps can contact the target region P in a case of the treatment, the insertion of the treatment probe 40 into the ultrasound probe 30 and the collection of the tissue can be achieved.

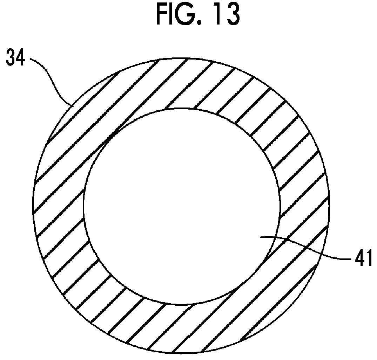

FIG. 12 is a diagram illustrating a second modification example of the treatment system 20, and is a diagram corresponding to FIG. 3. FIG. 13 is a schematic cross-sectional view taken along the line D-D in FIG. 12. In the modification example illustrated in FIG. 12, the configuration is the same as the configuration illustrated in FIG. 2, except that the hollow portion 31S of the tubular member 31 is closed at the distal end surface 312, a heat conduction member 34 is provided along the outer periphery at a side (in the example of FIG. 12, adjacent to the distal end surface 312 side) adjacent to the ultrasound transmission/reception unit 32 at the distal end part 31A of the tubular member 31, the treatment part 41 is connected to the heat conduction member 34, and the support member 55 is changed to a configuration in which the support member 55 is not movable in the axial direction of the tubular member 31.

As illustrated in FIG. 13, the heat conduction member 34 is formed in an annular shape, and an outer surface thereof is exposed. The treatment part 41 is inserted into the hollow portion of the heat conduction member 34 and is fixed to the heat conduction member 34 by fitting, adhesion, or the like.

In the modification example illustrated in FIG. 12, it is assumed that the treatment system 20 is sold in a state where the ultrasound probe 30, the operation handle 50, and the treatment probe 40 are assembled. However, the heat conduction member 34 and a portion of the tubular member 31 on the distal end side thereof may be configured as independent parts, and the treatment probe 40 may be inserted into the ultrasound probe 30 as illustrated in FIG. 11, and then this part may be fitted into the treatment part 41 to obtain the configuration of the modification example illustrated in FIG. 12.

A method of using the treatment system 20 of the modification example illustrated in FIG. 12 is the same as the method illustrated in FIG. 11. In the configuration illustrated in FIG. 12, in a case where the treatment part 41 is cooled, the cooling heat is transmitted to the heat conduction member 34, and the tissue around the heat conduction member 34 is frozen. Therefore, as in the configuration illustrated in FIG. 3 or FIG. 11, the tissue can be frozen and collected.

In FIG. 12, the heat conduction member 34 can be disposed adjacent to the proximal end side of the ultrasound transmission/reception unit 32, and the treatment part 41 can be connected to the heat conduction member 34. As illustrated in FIG. 12, with the configuration in which the heat conduction member 34 is provided between the distal end surface 312 and the ultrasound transmission/reception unit 32, even in a state where the ultrasound probe 30 and the treatment probe 40 protrude to the maximum from the treatment tool outlet port 10Ca, a freezing treatment can be accurately performed on the region observed by the ultrasound transmission/reception unit 32.

As described above, at least the following matters are described in the present specification. In the following, the components corresponding to the above-described embodiments are shown in parentheses, but the present disclosure is not limited thereto.
(1) A system (treatment system 20) comprising:
   a tubular member (tubular member 31) that has a hollow portion (hollow portion 31S) therein and that is provided, at one end part (distal end part 31A), with an ultrasound transducer (ultrasound transducer 32A) at an outer periphery of the hollow portion; and
   a device (operation handle 50) including a first support part (support member 55) that supports the other end part (proximal end part 31B) of the tubular member and a connection part (connection part 51) that is connectable to a treatment tool inlet port (treatment tool inlet port 112) of an endoscope (endoscope 1) in a state where the tubular member is inserted into the treatment tool inlet port from a side of the one end part,
   in which the tubular member has a second end surface on one end side (distal end surface 312) and a first end surface (proximal end surface 311) on the other end side in an axial direction, and has a first opening (proximal end opening 311A) connected to the hollow portion on at least the first end surface.
(2) The system according to (1),
   in which a second opening (distal end opening 312A) connected to the hollow portion is provided on the second end surface of the tubular member.
(3) The system according to (2),
   in which the device includes a second support part (support member 56) that supports a treatment tool (treatment probe 40) inserted into the hollow portion, and
   the first support part and the second support part support the tubular member and the treatment tool such that a relative position between a treatment part (treatment part 41) of the treatment tool and the second end surface of the tubular member is changeable.
(4) The system according to (3),
   in which the relative position includes a state where the treatment part protrudes through the second opening to an outside of the hollow portion and a state where at least a part of the treatment part is retracted from the second opening into the hollow portion.
(5) The system according to (4), further comprising:
   a first restriction part that restricts a protrusion amount of the treatment part from the hollow portion.
(6) The system according to any one of (1) to (5), further comprising:
   a treatment tool (treatment probe 40) inserted into the hollow portion,
   in which the treatment tool is capable of freezing tissue.
(7) The system according to any one of (2) to (5),
   in which the device includes a second support part (support member 56) that supports a treatment tool (treatment probe 40) inserted into the hollow portion, and
   the second support part supports the treatment tool in a state where a treatment part (treatment part 41) of the treatment tool protrudes through the second opening to an outside of the hollow portion.
(8) The system according to any one of (3) to (5) and (7),
   in which the device includes a moving mechanism that moves the tubular member and the treatment tool in the axial direction in a state of being connected to the treatment tool inlet port and in a state where the relative position between the second end surface and the treatment part is held.
(9) The system according to (8),
   in which the moving mechanism includes a second restriction part that restricts protrusion amounts of the tubular member and the treatment tool from a treatment tool outlet port (treatment tool outlet port 10Ca) on a distal end surface of the endoscope.
(10) The system according to any one of (1) to (5), further comprising:
   a treatment tool (treatment probe 40) inserted into the hollow portion,
   in which the treatment tool is capable of freezing tissue,
   a heat conduction member (heat conduction member 34) is provided at the one end part of the tubular member along an outer periphery at a side adjacent to the ultrasound transducer in the axial direction, and
   a treatment part (treatment part 41) of the treatment tool is connected to the heat conduction member.
(11) The system according to (10),
   in which the hollow portion of the tubular member is closed at the second end surface.
(12) The system according to (11),
   in which the heat conduction member is provided between the ultrasound transducer and the second end surface.
(13) The system according to any one of (1) to (12),
   in which a plurality of the ultrasound transducers are arranged along a circumferential direction of the one end part at an outer periphery of the tubular member at the one end part.
(14) A treatment method comprising:
   a first step of preparing a tubular member (tubular member 31) that has a hollow portion (hollow portion 31S) therein, the tubular member (tubular member 31) being provided, at one end part (distal end part 31A), with an ultrasound transducer (ultrasound transducer 32A) at an outer periphery of the hollow portion and having an opening (proximal end opening 311A) connected to the hollow portion on an end surface on the other end part (proximal end part 31B) side in an axial direction;
   a second step of inserting a treatment tool (treatment probe 40) into the hollow portion from the opening;
   a third step of inserting the tubular member in a state where the ultrasound transducer and a treatment part (treatment part 41) of the treatment tool are aligned into a subject and aligning the ultrasound transducer with a target region (target region P) of the subject based on an output of the ultrasound transducer;
   a fourth step of holding a position of the treatment part in a state where the target region, the ultrasound transducer, and the treatment part (treatment part 41) are aligned and moving the tubular member to the other end part (proximal end part 31B) side to expose the treatment part from the hollow portion; and
   a fifth step of driving the treatment part to treat the target region after the fourth step.
(15) The treatment method according to (14),
   in which the treatment tool is capable of freezing tissue at the treatment part, and
   the treatment method further comprises a sixth step of moving the treatment tool and the tubular member to collect the frozen tissue after the fifth step.

While various embodiments have been described above, it goes without saying that the present invention is not limited to such examples. It is apparent to those skilled in the art that various modifications and alterations can be conceived within the scope described in the claims, and such modifications are understood to fall within the technical scope of the invention. Further, the respective components of the embodiments described above may be combined arbitrarily without departing from the gist of the invention.

The present application is based on Japanese Patent Application (JP2023-169871) filed on September 29, 2023, the content of which is incorporated in the present application by reference.

### Explanation of References

1: endoscope
L1, L2: distance
1A: treatment tool channel
2: body part
4: light source device
4A: connector connection part
5: processor device
7: display device
10: insertion part
10A: flexible part
10B: bendable part
10C: endoscope distal end part
10Ca: treatment tool outlet port
11: operating part
11A: suction button
12: angle lever
13: universal cord
13A: connector part
20: treatment system
30: ultrasound probe
31: tubular member
31A: distal end part
31B: proximal end part
31S: hollow portion
31T, 40T: protrusion part
32: ultrasound transmission/reception unit
32A: ultrasound transducer
33: grip part
34: heat conduction member
40: treatment probe
41: treatment part
50: operation handle
51: connection part
52: shaft member
52a, 52b, 55a: flange part
53: slider
53K: opening
53a, 53b: step portion
53c: biasing portion
55, 56: support member
57: fixing member
100: endoscope device
112: treatment tool inlet port
112A: adapter
300: ultrasound probe control device
311: proximal end surface
311A: proximal end opening
312: distal end surface
312A: distal end opening
400: treatment probe control device

## Claims

1. A system comprising:
a tubular member that has a hollow portion therein and that is provided, at one end part, with an ultrasound transducer at an outer periphery of the hollow portion; and
a device including a first support part that supports other end part of the tubular member and a connection part that is connectable to a treatment tool inlet port of an endoscope in a state where the tubular member is inserted into the treatment tool inlet port from a side of the one end part,
wherein the tubular member has a second end surface on one end side and a first end surface on other end side in an axial direction, and has a first opening connected to the hollow portion on at least the first end surface.

2. The system according to claim 1,
wherein a second opening connected to the hollow portion is provided on the second end surface of the tubular member.

3. The system according to claim 2,
wherein the device includes a second support part that supports a treatment tool inserted into the hollow portion, and
the first support part and the second support part support the tubular member and the treatment tool such that a relative position between a treatment part of the treatment tool and the second end surface of the tubular member is changeable.

4. The system according to claim 3,
wherein the relative position includes a state where the treatment part protrudes through the second opening to an outside of the hollow portion and a state where at least a part of the treatment part is retracted from the second opening into the hollow portion.

5. The system according to claim 4, further comprising:
a first restriction part that restricts a protrusion amount of the treatment part from the hollow portion.

6. The system according to any one of claims 1 to 5, further comprising:
a treatment tool inserted into the hollow portion,
wherein the treatment tool is capable of freezing tissue.

7. The system according to claim 2,
wherein the device includes a second support part that supports a treatment tool inserted into the hollow portion, and
the second support part supports the treatment tool in a state where a treatment part of the treatment tool protrudes through the second opening to an outside of the hollow portion.

8. The system according to any one of claims 3 to 5 and 7,
wherein the device includes a moving mechanism that moves the tubular member and the treatment tool in the axial direction in a state of being connected to the treatment tool inlet port and in a state where the relative position between the second end surface and the treatment part is held.

9. The system according to claim 8,
wherein the moving mechanism includes a second restriction part that restricts protrusion amounts of the tubular member and the treatment tool from a treatment tool outlet port on a distal end surface of the endoscope.

10. The system according to claim 1, further comprising:
a treatment tool inserted into the hollow portion,
wherein the treatment tool is capable of freezing tissue,
a heat conduction member is provided at the one end part of the tubular member along an outer periphery at a side adjacent to the ultrasound transducer in the axial direction, and
a treatment part of the treatment tool is connected to the heat conduction member.

11. The system according to claim 10,
wherein the hollow portion of the tubular member is closed at the second end surface.

12. The system according to claim 11,
wherein the heat conduction member is provided between the ultrasound transducer and the second end surface.

13. The system according to any one of claims 1 to 5 and 10 to 12,
wherein a plurality of the ultrasound transducers are arranged along a circumferential direction of the one end part at an outer periphery of the tubular member at the one end part.
